# EUROPÄISCHE PATENTSCHRIFT

(11) **EP 1 127 056 B1**
(45) Veröffentlichungstag und Bekanntmachung des Hinweises auf die Patenterteilung: **11.12.2002**
(21) Anmeldenummer: 99955889.3
(22) Anmeldetag: 27.10.1999
(51) Int. Cl.: C07D 317/42

(54) **VERFAHREN ZUR HERSTELLUNG VON MONOHALOGENIERTEN 2-OXO-1,3-DIOXOLANEN**
METHOD FOR PRODUCING MONOHALOGENATED 2-OXO-1,3-DIOXOLANES
PROCEDE POUR LA PRODUCTION DE 2-OXO-1,3-DIOXOLANNES MONOHALOGENES

(30) Priorität: 05.11.1998 DE 19850906
(43) Veröffentlichungstag der Anmeldung: 29.08.2001
(73) Patentinhaber: MERCK PATENT GmbH, 64293 Darmstadt (DE)
(72) Erfinder: SCHUHMANN, Rosemarie, 33803 Steinhagen (DE)
(86) Internationale Anmeldenummer: EP9908113
(87) Internationale Veröffentlichungsnummer: WO00027837

(56) Entgegenhaltungen:
- DE-B- 1 203 796
- US-A- 2 918 478
- CHEMICAL ABSTRACTS, vol. 131, no. 5, 1999 Columbus, Ohio, US; abstract no. 58816k, Seite 659; Spalte 1; XP002130667 & JP 11 171882 A (MITSUBISHI CHEM. IND.) 29. Juni 1999 (1999-06-29)

## Beschreibung

Die Erfindung betrifft ein Verfahren zur Herstellung von monohalogenierten 2-Oxo-1,3-dioxolanen der allgemeinen Formel

C₃H₃XO₃ (I)

worin
- X: Cl oder Br
bedeutet, dadurch gekennzeichnet, daß die Monohalogenierung ausgehend von Ethylencarbonat mit Sulfuryldihalogeniden unter UV-Bestrahlung und in Abwesenheit von Lösungsmitteln durchgeführt wird.

Halogenierte 2-Oxo-1,3-dioxolane werden in elektrochemischen Zellen den Lösungsmitteln zugesetzt. Sie dienen der Erhöhung der Stabilität der Elektrodenmaterialien. Zudem sind sie Zwischenprodukte für die Herstellung von Vinylencarbonat (II), das als Zusatz in Elektrolytlösungen für elektrochemische Zellen eingesetzt wird. Das Vinylencarbonat wirkt als Stabilisator der Elektrodenmaterialien. Vinylencarbonate werden über eine Eliminierungsreaktion aus den monohalogenierten 2-Oxo-1,3-dioxolanen (I) oder auch über die Reaktion von dihalogenierten Verbindungen (III) mit Zn, wie von M.S. Newman in J. Am. Chem. Soc., 77, 3789-3793, (1955) beschrieben, gewonnen.

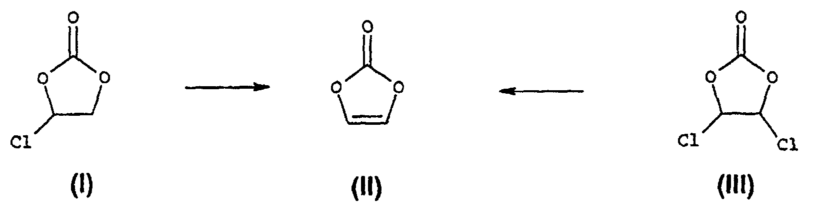

Chlorierte 2-Oxo-1,3-dioxolane wie 4-Chlor-2-oxo-1,3-dioxolan (I) werden üblicherweise durch Chlorierung der entsprechenden 2-Oxo-1,3-dioxolane (IV) dargestellt. In US 3,021,340 wird Ethylencarbonat (2-Oxo-1,3-dioxolan) in Tetrachlorkohlenstoff und wasserfreiem Eisenchlorid bis zum Siedepunkt erhitzt. Durch die Lösung wird 36 Stunden lang Chlor geleitet. Die destillative Aufbereitung ergibt eine Mischung aus mono- und dichloriertem Dioxolan (I) und (III).

Die Veränderung der Reaktionsbedingungen, wie zum Beispiel die Bestrahlung der Reaktionslösung mit einer 350-W-Lampe führt zu einer wesentlichen Verkürzung der Reaktionszeit (R.G. Finke et al., J. Am. Chem. Soc. (105), 7592-7604, (1983)). Auch bei diesem Verfahren wird neben 4-Chlor-2-oxo-1,3-dioxolan (I) das 4,5-Dichloro-2-oxo-1,3-dioxolan (III) gebildet.

Die Chlorierung kann nicht nur mit Chlor durch Bestrahlung mit Licht geeigneter Wellenlänge durchgeführt werden, sondern auch durch den Einsatz von Substanzen, die unter Bildung freier Radikale zerfallen. Es wurde beobachtet, daß bei der Verwendung von Sulfurylchlorid als Chlorierungsmittel geringe Mengen organischer Peroxide ähnlich aktivierende Eigenschaften besitzen wie Licht (M.S. Kharasch et al., Am. Soc., (61), 2142, (1939); (62), 925 ff., (1940)). In G. Wulff et al., Chem. Ber. (125), 473-477, (1992) wird zur Darstellung von chlorierten 2-Oxo-1,3-dioxolanen Sulfurylchlorid verwendet. Die Lösung wird in Tetrachlorkohlenstoff erhitzt und mit einer 500-W-Lampe bestrahlt. Das mono- und das dichlorierte Dioxolan werden isoliert.

Der Einsatz von Tetrachlorkohlenstoff als Lösungsmittel erfordert erhöhte Sicherheitsvorkehrungen, da dieser Stoff als giftig, gesundheitsschädlich, irreversibel schädigend und umweltgefährlich eingestuft ist.

Aufgabe der Erfindung war es deshalb, ein umweltschonendes, preiswertes und selektiv die monochlorierte Verbindung darstellendes Verfahren zu entwickeln.

Die erfindungsgemäße Aufgabe wird gelöst durch ein neues Verfahren zur Herstellung von monohalogenierten 2-Oxo-1,3-dioxolanen der allgemeinen Formel

C₃H₃XO₃ (I)

worin
- X: Cl oder Br
bedeutet, dadurch gekennzeichnet, daß die Monohalogenierung ausgehend von Ethylencarbonat mit Sulfuryldihalogeniden unter UV-Bestrahlung, bei Temperaturen zwischen 0°C und 90°C, in Abwesenheit von Lösungsmitteln, unter Atmosphärendruck und Anwesenheit von Luftsauerstoff oder gegebenenfalls unter Schutzgasatmosphäre durchgeführt wird.

Durch Versuche wurde nun gefunden, daß die Reaktion der Ausgangsverbindung 2-Oxo-1,3-dioxolan (IV) mit Sulfurylchlorid ohne Tetrachlorkohlenstoff durchgeführt werden kann.

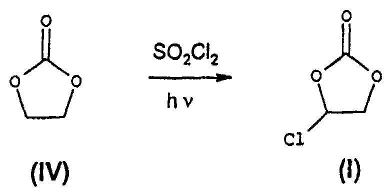

Die Reaktionsgeschwindigkeit erwies sich hierbei nur geringfügig niedriger als bei der Synthese mit Tetrachlorkohlenstoff (CCl₄). Überraschenderweise wurde dabei gefunden, daß bei Einsatz von equimolaren Mengen Ethylencarbonat und Sulfurylchlorid im erfindungsmäßigen Verfahren die Bildung des unerwünschten dichlorierten Nebenproduktes vermieden wird, wenn in Abwesenheit von Tetrachlorkohlenstoff gearbeitet wird.

Zusätzlich vereinfacht sich die Aufarbeitung, da weder das Lösungsmittel noch Dichlorethylencarbonat destillativ entfernt werden müssen. Bei einer weiteren Umsetzung zu Vinylencarbonat kann sogar ganz auf eine vorherige Aufarbeitung verzichtet werden, da das nicht umgesetzte Ethylencarbonat problemlos nach diesem weiteren Reaktionsschritt destillativ abgetrennt werden kann.

Ein besonderer Vorteil des erfindungsgemäßen Verfahrens ist, daß trotz der Abwesenheit des Lösungsmittels eine hohe Reaktionsgeschwindigkeit und hohe Quantenausbeute erzielt werden. Bei der Durchführung des erfindungsgemäßen Verfahrens wurde auch gefunden, daß die Gasentwicklung, d.h. die Bildung von HCI und SO₂, durch die Strahlungsintensität reguliert werden kann. Dies erleichtert die Verfahrensführung erheblich, da die Notwendigkeit große Gasmengen in kürzester Zeit abführen zu müssen, vermieden werden kann. Durch die wesentlich verlangsamte Gasentwicklung kann auf die chargenweise Zuführung der Edukte verzichtet werden, und es muß auch nicht auf ein Abklingen der Gasentwicklung gewartet werden. Es bietet sich für ein Up-Scaling der Reaktion neben der Batch-Fahrweise eine kontinuierliche Reaktionsführung an. Von Vorteil ist dabei die geringe Verweilzeit aufgrund der hohen Reaktionsgeschwindigkeit. Die kontinuierliche Reaktionsführung kann beispielsweise durch eine Kaskade mehrerer konventioneller Photoreaktoren oder Dünnschichtrollenphotoreaktoren durchgeführt werden. Durch den Verzicht auf Tetrachlorkohlenstoff als Lösungsmittel wird das Genehmigungsverfahren für eine großtechnische Anlage wesentlich vereinfacht. Die Umsetzung muß entgegen bisheriger Durchführungsvorschriften nicht unter Schutzgasatmosphäre durchgeführt werden. Die Einsparung von Rohstoffen, z.B. der Lösungsmittel und des Schutzgases, sowie der dadurch wesentlich vereinfachte Apparaturaufbau führen zu erheblich verringerten Kosten.

Zur Herstellung von monohalogenierten 2-Oxo-1,3-dioxolanen wird eine kühlbare Apparatur mit einer entsprechenden Vorrichtung zur Temperaturüberwachung im Reaktionsgefäß, einer Gaszu- und ableitung, eine Mischvorrichtung sowie einer Vorrichtung zur Erzeugung von UV-Strahlung, wie z.B. eine Quecksilberhochdruckdampflampe, benötigt. Der Apparatur können Einrichtungen zur Gaswäsche, zur Absorption von HCI und SO₂ nachgeschaltet werden.

Zur Durchführung des erfindungsgemäßen Verfahrens werden in der Apparatur Ethylencarbonat und Sulfuryldihalogenide vorgelegt und unter Atmosphärendruck bis leichtem Überdruck und in Anwesenheit von Luftsauerstoff oder gegebenenfalls unter Schutzgasatmosphäre belichtet. Die Temperatur im Reaktionsgefäß wird mit Eiswasserkühlung oder einem Kryostaten u.ä. zwischen 0°C und 70°C gehalten. Eine gute Reaktionskinetik wird bei Temperaturen zwischen 20°C und 70°C festgestellt. Die Belichtungszeit ist variabel und liegt, in Abhängigkeit von der Intensität der Strahlung und dem gewünschtem Umsatz, zwischen 15 und 300 min. Bei chargenweiser Zugabe des Sulfurylchlorids und einer Belichtungszeit von 30 min wurde eine Ausbeute an monohalogeniertem 2-Oxo-1,3-dioxolan von 79% erzielt. Eine kontinuierliche, equimolare Zudosierung von Sulforylhalogenid bietet den Vorteil, daß die Gasentwicklung damit kontrollierbar erfolgt. Damit kann die Belichtungszeit auf 15 bis 60 min reduziert werden. Dem Fachmann ist es damit möglich, analog den eingesetzten Mengen an Edukten, die entsprechenden Parameter wie Temperatur und Belichtungszeit optimal auszuwählen. Überraschend wurden bei equimolarer Reaktionsführung keine Spuren eines dihalogenierten Nebenproduktes gefunden. Die destillative Aufreinigung vereinfacht sich, da lediglich nicht umgesetztes Ausgangsmaterial, aber nicht Nebenprodukte und Lösungsmittel entfernt werden müssen.

Die im folgenden gegebenen Beispiele werden zur besseren Veranschaulichung der vorliegenden Erfindung gegeben, sind jedoch nicht dazu geeignet, die Erfindung auf die hierin offenbarten Merkmale zu beschränken.

### Beispiele

### Beispiel 1:

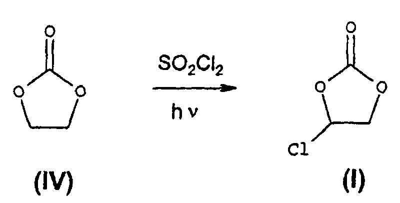

In einer kühlbaren Apparatur mit Quecksilberhochdruckdampflampe, Thermometer, Rührer, Gaszu- und ableitung und nachgeschalteter Gaswaschflaschen mit wäßriger Natronlauge werden 80 g Ethylencarbonat und 73,6 ml Sulfurylchlorid unter Rühren vorgelegt. Es wird nicht unter Schutzgasatmosphäre gearbeitet. Die photolytische Zersetzung von Sulfurylchlorid wird durch Einschalten der Quecksilberhochdruckdampflampe gestartet. Die Reaktionslösung wird durch Wasserkühlung auf einer Temperatur zwischen 20°C und 40°C gehalten. Nach 15, 30 und 60 min Belichtungszeit werden Proben für kinetische Untersuchungen entnommen. Die Ergebnisse sind in der Tabelle 1 zusammengestellt.

**Tabelle 1:**

| Photolytische Umsetzung von Ethylencarbonat mit Sulfurylchlorid ohne Lösungsmittel | | |
|---|---|---|
| Belichtungsdauer [min] | Umsatz [%] | Verhältnis Chlorethylencarbonat : Dichlorethylencarbonat |
| 15 | 61 | 100 : 0 |
| 30 | 79 | 100 : 0 |
| 60 | 79 | 100 : 0 |

Durch die Abwesenheit von Lösungsmittel und des unerwünschten Nebenproduktes Dichlorethylencarbonat kann auf eine destillative Aufarbeitung verzichtet werden.

### Vergleichsbeispiel 1:

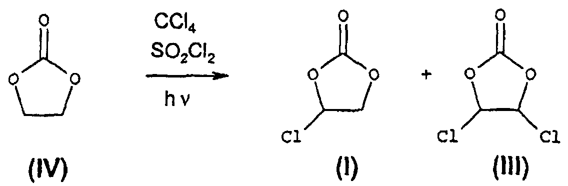

In einer kühlbaren Apparatur mit Quecksilberhochdruckdampflampe, Thermometer, Rührer, Gaszu- und ableitung und nachgeschalteter Gaswaschflaschen mit wäßriger Natronlauge werden unter Argonatmosphäre 80,0 g Ethylencarbonat, 73,6 ml Sulfurylchlorid und 300 ml Tetrachlorkohlenstoff unter Rühren vorgelegt. Es liegt ein Zweiphasengemisch vor, wobei das Carbonat nur geringfügig in der CCl₄-Phase löslich ist. Die photolytische Zersetzung von Sulfurylchlorid wird durch Einschalten der Quecksilberhochdruckdampflampe gestartet, wobei sofort eine heftige Gasentwicklung einsetzt. Die Reaktionsmischung wird durch Wasserkühlung auf Temperaturen zwischen 20°C und 40°C gehalten. Nach 15 und 60 min Belichtungszeit werden Proben für kinetische Untersuchungen entnommen. Die Ergebnisse sind in Tabelle 2 zusammengestellt.

**Tabelle 2:**

| Photolytische Umsetzung von Ethylencarbonat mit Sulfurylchlorid in Tetrachlorkohlenstoff | | |
|---|---|---|
| Belichtungsdauer [min] | Umsatz [%] | Verhältnis Chlorethylencarbonat : Dichlorethylencarbonat |
| 15 | 74 | 96 : 4 |
| 60 | 84 | 94 : 6 |

Nach dem Abkühlen des Reaktionsgemisches auf Raumtemperatur werden bei Normaldruck Reste von CCl₄ abdestilliert und das Rohprodukt anschließend im Ölpumpenvakuum von 7 Torr bei einem Übergangstemperaturintervall von 50 bis 88°C destilliert. Es werden 88,8 g eines hellgelben Öls, bestehend aus 78% Chlorethylencarbonat, 6% Dichlorethylencarbonat und 16% Ethylencarbonat, isoliert.

## Patentansprüche

1. Verfahren zur Herstellung von monohalogenierten 2-Oxo-1,3-dioxolanen der allgemeinen Formel
C₃H₃XO₃ (I)
worin
X Cl oder Br
bedeutet, **dadurch gekennzeichnet, daß** die Monohalogenierung ausgehend von Ethylencarbonat mit Sulfuryldihalogeniden unter UV-Bestrahlung, bei Temperaturen zwischen 0°C und 70°C, in Abwesenheit von Lösungsmitteln, unter Atmosphärendruck und Anwesenheit von Luftsauerstoff oder gegebenenfalls unter Schutzgasatmosphäre durchgeführt wird.

2. Verfahren gemäß Anspruch 1, **dadurch gekennzeichnet, daß** die Monohalogenierung bei Temperaturen zwischen 20°C und 70°C durchgeführt wird.

3. Verfahren gemäß Anspruch 1, **dadurch gekennzeichnet, daß** die Monohalogenierung unter Schutzgasatmosphäre durchgeführt wird.

## Claims

1. Process for the preparation of monohalogenated 2-oxo-1,3-dioxolanes of the general formula
C₃H₃XO₃ (I)
in which
X is Cl or Br,
**characterized in that** the monohalogenation is carried out starting from ethylene carbonate with sulfuryl halides under UV irradiation, at temperatures between 0°C and 70°C, in the absence of solvents, under atmospheric pressure and in the presence of atmospheric oxygen or optionally under a protective-gas atmosphere.

2. Process according to Claim 1, **characterized in that** the monohalogenation is carried out at temperatures between 20°C and 70°C.

3. Process according to Claim 1, **characterized in that** the monohalogenation is carried out under a protective-gas atmosphere.

## Revendications

1. Procédé pour la préparation des 2-oxo-1,3-dioxanes monohalogénés de formule générale
C₃H₃XO₃ (I)
où
X représente Cl ou Br,
**caractérisé en ce que** l'on effectue la monohalogénation en partant du carbonate d'éthylène avec des dihalogénures de sulfuryle par traitement par les radiations UV, à des températures comprises entre 0°C et 70°C, en l'absence de solvants sous la pression atmosphérique, et en présence de l'oxygène de l'air ou éventuellement sous atmosphère de gaz protecteur.

2. Procédé selon la revendication 1, **caractérisé en ce que** l'on effectue la monohalogénation à des températures comprises entre 20°C et 70°C.

3. Procédé selon la revendication 1, **caractérisé en ce que** l'on effectue la monohalogénation sous atmosphère de gaz protecteur.
